# EUROPEAN PATENT SPECIFICATION

(11) **EP 2 929 039 B1**
(45) Date of publication and mention of the grant of the patent: **24.07.2019**
(21) Application number: 13862320.2
(22) Date of filing: 10.12.2013
(51) Int. Cl.: C12P 7/40, C12P 7/56

(54) **BATCH FEED PROCESS FOR FERMENTING SUGARS**
CHARGENZUFUHRPROZESS ZUR FERMENTIERUNG VON ZUCKERN
PROCÉDÉ D'ALIMENTATION DISCONTINUE POUR FERMENTATION DE SUCRES

(30) Priority: 10.12.2012 US 201261735448 P
(43) Date of publication of application: 14.10.2015
(73) Proprietor: Cargill, Incorporated, Wayzata, MN 55391 (US)
(72) Inventor: GOKARN, Ravi, Omaha, Nebraska 68130 (US); PARSONS, Matthew, Blair, Nebraska 68008 (US); WALCKER, Derran, Omaha, Nebraska 68134 (US); SPENCER, Joseph, Bennington, Nebraska 68007 (US)
(74) Representative: Fox, Charlotte
(86) International application number: PCT/US2013/074070
(87) International publication number: WO 2014/093312

(56) References cited:
- EP-A1- 2 241 632
- WO-A2-2004/009827
- WO-A2-2004/018645
- WO-A2-2007/057018
- US-A- 5 853 487
- US-A1- 2009 011 481
- US-A1- 2009 011 481
- US-B1- 8 415 136
- SIEW LING HII ET AL: "Pullulanase: Role in Starch Hydrolysis and Potential Industrial Applications", ENZYME RESEARCH, vol. 2012, 921362, 12 June 2012 (2012-06-12), pages 1-14, XP055282385, DOI: 10.1155/2012/921362
- TAMBURINI, E. ET AL.: 'Near-Infrared Spectroscopy: A Tool for Monitoring Submerged Fermentation Processes Using an Immersion Optical-Fiber Probe.' APPLIED SPECTROSCOPY. vol. 57, no. 2, 01 January 2003, pages 132 - 138, XP055260247
- FUJIWARA, E ET AL.: 'Real-Time Monitoring of Fermentation Process Applied to Sugarcane Bioethanol Production.' P ROC. SPIE 8421, OFS2012 22ND INTERNATIONAL CONFERENCE ON OPTICAL FIBER SENSORS, 842164. 04 October 2012, pages 1 - 4, XP055260255 DOI: 10.1117/12.970511

## Description

### FIELD OF THE INVENTION

The present invention relates to a process for fermenting sugars. More particularly, the present invention relates to a batch feed process for fermenting sugars.

### BACKGROUND OF THE INVENTION

Fermentation processes are used commercially at large scale to produce organic molecules such as ethanol, citric acid and lactic acid. In those processes, a carbohydrate is fed to a organism that is capable of metabolizing it to the desired fermentation product. The carbohydrate and organism are selected together so that the organism is capable of efficiently digesting the carbohydrate to form the product that is desired in good yield. It is becoming more common to use genetically engineered organisms in these processes, in order to optimize yields and process variables, or to enable particular carbohydrates to be metabolized.

Starch is a widely available and inexpensive carbohydrate source. It is available from a wide variety of plant sources such as corn, wheat, rice, barley, and the like. Many organisms are not capable of metabolizing starch directly, or else metabolize it slowly and inefficiently. Accordingly, it is common to treat starch before feeding it into the fermentation process, in order to break it down into monosaccharides that the organism can ferment easily.

Usually, starch is hydrolyzed to form a mixture containing mainly glucose (i.e., dextrose). However, complete hydrolysis to glucose adds significant cost, so most commercially available glucose products tend to contain a small amount of fructose and various oligomeric polysaccharides. Unfortunately, many organisms cannot metabolize the oligomers, either, and so these carbohydrate values are wasted.

The published patent application US2009/011481 discloses a fermentation process for fermenting a starch hydrolysate, which comprises a step of adding to the fermentation broth an effective quantity of at least one enzyme that depolymerises at least one oligomeric saccharide to glucose and also discloses transglucosidase enzymes which convert isomaltose into glucose.

### SUMMARY OF THE INVENTION

Isomaltose is a component that may be formed during the process of preparing starch hydrolysate raw materials having high glucose concentrations. It has been found that isomaltose is a particularly undesirable component to be present, particularly at the later stages of the present process to ferment glucose from a starch hydrolysate source to form fermentation products. While not being bound by theory, it is believed that isomaltose may interfere with the product yield not only by preventing fermentation of the two constituent glucose monomer components of the isomaltose, but also by reacting with the final fermentation products. The isomaltose may react with the fermentation products to form for example ester and other chemicals, which are formed via the reaction of a plurality of available hydroxy functionalities of the isomaltose. For example, if the product is an amino acid, the isomaltose may react with the product via the Maillard reaction to produce chemical compounds which cause browning. Thus each isomaltose compound present at the end of the fermentation process has a multiplier effect in reducing the yield and/or quality of the desired final fermentation product.

It has further been found that addition of an enzyme to a starch hydrolysate material having high glucose concentration tends to render the enzyme less effective. The present invention provides an efficient and elegant way to ferment starch hydrolysates having high glucose concentration. The present process is particularly beneficial for the preparation of fermentation products to be used in non-food applications. Surprisingly fermentation products used in non-food applications require a different standard of purity, because some residual ingredients of fermentation processes that may be present in food products are not acceptable in non-food applications.

In a first embodiment, a batch fermentation process is provided that ferments a starch hydrolysate containing 80-98 weight percent of glucose based on total carbohydrate and 0.3-5% weight percent of isomaltose based on total carbohydrate to a fermentation product. In this process, a fermentation broth is formed containing a first portion of a total amount of the starch hydrolysate so that the fermentation broth has an initial glucose concentration of at least 50 g/L. The starch hydrolysate in the fermentation broth is fermented in an initial fermentation step to produce a fermentation product until the fermentation broth contains 30 g/L or less of glucose. After achieving this glucose concentration, an effective amount of at least one active enzyme that converts isomaltose into glucose is added to the fermentation broth. Then the remaining portion of the total amount of starch hydrolysate containing 80-98 weight percent of glucose is fed into the fermentation broth to maintain a glucose concentration of from 5 to 15 g/L in the fermentation broth throughout the feeding step. The final fermentation broth containing the fermentation product is then produced.

In a second embodiment, the glucose concentration is monitored by a real-time monitoring system, and the real-time monitoring system interfaces with equipment that control the introduction of starch hydrolysate into the fermentation broth. In this second embodiment, the fermentation process is a batch fermentation process which utilizes a starch hydrolysate containing at least 60 weight percent of glucose based on total carbohydrate and 0.3-5% weight percent of isomaltose based on total carbohydrate to produce a final fermentation broth containing the fermentation product. In this second embodiment, a fermentation broth is formed containing a first portion of a total amount of the starch hydrolysate so that the fermentation broth has an initial glucose concentration of at least 50 g/L, the starch hydrolysate in the fermentation broth is fermented in an initial fermentation step to produce a fermentation product until the fermentation broth contains 30 g/L or less of glucose, then an effective amount of at least one active enzyme that converts isomaltose into glucose is added to the fermentation broth, and subsequently the remaining portion of the total amount of starch hydrolysate containing at least 60 weight percent of glucose based on total carbohydrate and 0.3-5% weight percent of isomaltose based on total carbohydrate is fed into the fermentation broth to maintain a glucose concentration of from 5 to 15 g/L in the fermentation broth throughout the feeding step. Finally, the feed of starch hydrolysate is ceased and the fermentation is allowed to continue until the final fermentation broth is produced.

### DETAILED DESCRIPTION OF PRESENTLY PREFERRED EMBODIMENTS

The embodiments of the present invention described below are not intended to be exhaustive or to limit the invention to the precise forms disclosed in the following detailed description. Rather a purpose of the embodiments chosen and described is so that the appreciation and understanding by others skilled in the art of the principles and practices of the present invention can be facilitated.

In the present batch fermentation process, a starch hydrolysate is used as the starting material. The starch may be obtained from any suitable starch source, such as corn, wheat, rice, barley, potatoes, cassava, and the like. The starch hydrolysates used in the present fermentation process are preferably obtained from a starch that has undergone a liquefaction and saccharification in order to obtain a high concentration of glucose. The balance of carbohydrate in such hydrolysates are composed of varying concentrations of fructose, maltose, isomaltose, panose, and other DP3 and DP4 (i.e. oligomers of glucose) or greater glucose oligomers. Such starch hydrolysates are a particularly preferred fermentation substrate for use in this invention. In the present process, the starch hydrolysate to be used in the fermentation process contains 80-98 weight percent of glucose based on total carbohydrate and 0.3-5 weight percent of isomaltose based on total carbohydrate (for example, from 0.4-5 weight percent, from 0.5-3 weight percent, and from 0.7-2.5 weight percent isomaltose based on total carbohydrate). Optionally, the starch hydrolysate to be used in the fermentation process contains 85-98 weight percent of glucose based on total carbohydrate or 90-97 wt% of glucose based on the total carbohydrate. Optionally, the starch hydrolysate to be used in the fermentation process contains 0.5-3% weight percent of isomaltose based on total carbohydrate (for example, from 0.4-5 weight percent, from 0.5-3 weight percent, and from 0.7-2.5 weight percent isomaltose based on total carbohydrate), or in some instances where higher level of isomaltose are present from 1-2 percent by weight isomaltose based on the total carbohydrate. Optionally, at least about 90% by weight of the solids content of the starch hydrolysate is carbohydrate. Optionally, at least about 95% by weight of the solids content of the starch hydrolysate is carbohydrate. Typically at least about 97% by weight of solids content (for example at least about 98 % by weight of solids content of the starch hydrolysate is carbohydrate) and in some instances at least 99% by weight of solids content of the starch hydrolysate is carbohydrate. Optionally, the starch hydrolysate is provided as a starting material composition having a dry solids content of from about 20 to about 70%. Optionally, the starch hydrolysate is provided as a starting material composition having a dry solids content of from about 25 to about 45%. Optionally, the starch hydrolysate is provided as a starting material composition having a dry solids content of from about 50 to about 70%. Starch hydrolysates having higher solids content can be easier and less expensive to ship than those which have lower solids content, which provides particular benefit for applications where the facility for carrying out the starch hydrolysis is some distance away from the facility for carrying out the batch fermentation process.

A fermentation broth is formed containing a first portion of a total amount of the starch hydrolysate so that the fermentation broth has an initial glucose concentration of at least 50 g/L. The fermentation broth is prepared by mixing the starch hydrolysate, water, and nutrients necessary to support growth of the fermentation organism and inoculating with an organism suitable for carrying out the desired fermentation. Optionally, the initial glucose concentration is from 50 g/L to about 170 g/L. Optionally, the initial glucose concentration is from about 70 g/L to about 140 g/L. Optionally, the initial glucose concentration is from about 80 g/L to about 130 g/L. Optionally, the initial glucose concentration is from about 90 g/L to about 120 g/L.

Embodiments using a higher initial concentration of glucose in the fermentation broth are particularly advantageous, because one may take advantage of the better economics that come with using higher starting concentrations of starting materials.

The organism used in the present fermentation is one which can ferment glucose to the desired fermentation product. As such, the particular organism used is selected in relation to the fermentation product that is desired. The organism may be naturally occurring, or may be a mutant or recombinant strain. Examples of organisms include various species of bacilli, lactobacilli, filamentous fungi, and yeast, including wild-types and mutated or recombinant types. Suitable organisms useful for producing lactic acid include wild-type bacteria from the genera Lactobacillus, Pediococcus, Lactococcus and Streptococcus and wild-type fungi of the genera Rhizopus. In addition, recombinant yeast strains of the genera Kluyveromyces, Pichia, Hansenula, Candida, Trichosporon, Issatchenkia, or Yamadazyma, having exogenous LDH genes are suitable. Such recombinant yeast strains are described in WO 99/14335, WO 00/71738 and WO 02/42471, for example. Additional organisms include fungi, algae and archaea. Mixtures of organisms are also specifically contemplated.

The fermentation broth will also include water and preferably includes nutrients, such as proteins (or other nitrogen source), vitamins and salts, which are necessary or desirable for cell function. Other components may also be present in the fermentation broth, such as buffering agents, fermentation products (which tend to accumulate as the fermentation progresses), and other metabolites. In cases where the fermentation is an acid, it is common to buffer the broth with a base such as calcium hydroxide or calcium carbonate, ammonia or ammonium hydroxide, sodium hydroxide, or potasium hydroxide in order to maintain a pH at which the organism functions well.

The present batch fermentation process adds starch hydrolysate at two separate times in the fermentation process in order to ferment a total amount of starch hydrolysate. The first portion is added before fermentation is conducted to reach the desired glucose concentration and addition of the enzyme. The first portion of the total amount of the starch hydrolysate preferably contains from about 50 to about 80 percent of the total amount of starch hydrolysate to be added to the fermentation broth.

The starch hydrolysate is then fermented in the fermentation broth in an initial fermentation step to produce a fermentation product until the fermentation broth contains 30 g/L or less of glucose. Optionally, the starch hydrolysate is fermented until the fermentation broth contains about 25 g/L or less of glucose in the initial fermentation step. Optionally, the starch hydrolysate is fermented until the fermentation broth contains about 20 g/L or less of glucose. Optionally, the starch hydrolysate is fermented until the fermentation broth contains about 15 g/L or less of glucose. Optionally, a minimum amount of glucose is desirable in the system to assure that the fermentation process can continue to operate without interruption during the fermentation process. Thus, optionally, the starch hydrolysate is fermented until the fermentation broth contains from 30 g/L to about 5 g/L of glucose, or from about 25 g/L to about 5 g/L of glucose, or from about 20 g/L to about 5 g/L of glucose, or from about 15 g/L to about 5 g/L of glucose. Similarly, optionally, the starch hydrolysate is fermented until the fermentation broth contains from 30 g/L to about 8 g/L of glucose, or from about 25 g/L to about 8 g/L of glucose, or from about 20 g/L to about 8 g/L of glucose, or from about 15 g/L to about 8 g/L of glucose. Similarly, optionally, the starch hydrolysate is fermented until the fermentation broth contains from 30 g/L to about 10 g/L of glucose, or from about 25 g/L to about 10 g/L of glucose, or from about 20 g/L to about 10 g/L of glucose, or from about 15 g/L to about 10 g/L of glucose.

The fermentation is carried out under conditions so that fermentation can occur. Although conditions can vary depending on the particular organism and desired fermentation product, typical conditions include a temperature of from about 20°C., preferably from about 30°C. to about 50°C., more preferably about 45°C. Usually the reaction mixture is mixed. The mixing may occur by sparging gas to the fermentation broth or alternatively via direct mechanical agitation or by other means.

Once this above described concentration of glucose is achieved during the initial fermentation step, an effective amount of at least one active enzyme that converts isomaltose into glucose is adding to the fermentation broth. The enzyme may be any enzyme effective for depolymerizing isomers having a 1,6 ether linkage. A preferred enzyme for acting on isomers having 1,6 linkages is trans-glucosidase.

The amount of enzyme to be used depends somewhat on the particular enzyme, the desired rate of reaction, and presence of any other oligomeric polysaccharide(s) that may also be effectively depolymerized by the enzyme. Enzyme activity is commonly measured in Units (U) of activity. One unit of enzyme activity is defined as the amount of enzyme activity that will convert one micro-mole of substrate per minute. Optionally, the enzyme is used in a quantity sufficient to provide about 0.05- 5 Units of enzyme activity for an isomaltose substrate per liter of fermentation broth. Optionally, the quantity of enzyme is from about 0.1- 3 Units of enzyme activity per liter of fermentation broth, Optionally, the quantity of enzyme is from about about 0.3- 1 Units of enzyme activity per liter of fermentation broth Similarly, these Units of enzyme activity per liter of fermentation broth may be used to determine the amount of enzyme suitable for conversion of other oligomeric polysaccharide(s) that may be present in the fermentation broth. The above number of Units is used to provide a good approximation of the amount of enzyme to be added to the fermentation broth, which is evaluated by experimental observation to determine actual amounts of enzyme required under conditions of use (e.g. temperature, pH and other conditions particular to a given fermentation process) to achieve the desired concentrations of residual oligosaccharides in the final fermentation broth at the lowest cost of enzymes.

The step of adding to the fermentation broth an effective amount of at least one active enzyme that converts isomaltose into glucose is carried out under conditions typically to obtain the conversion of the isomaltose and the fermentation of the glucose occur simultaneously. Optionally, the active enzyme is added to the fermentation broth in a single addition step. In an embodiment, the active enzyme is added to the fermentation broth in a single addition step metered over a time of from 3 minutes to 3 hours. Optionally, the active enzyme is added to the fermentation broth in a plurality of addition steps after the initial fermentation step. Optionally, at least a portion of the enzyme is added after the remaining portion of the starch hydrolysate is fed, and the amount of isomaltose present in the fermentation broth is monitored and additional enzyme is added as needed to achieve the desired isomaltose level.

Addition of the enzyme after the initial fermentation step, once the glucose concentration level is achieved, maximizes its effectiveness, because it has been found that high glucose concentration compositions tend to experience reverse conversion of glucose to isomaltose and additionally that the enzyme itself is inhibited by high concentration of glucose. Further, the enzyme has a finite time of effectiveness, and so later addition to the fermentation process is advantageous to use the enzyme during its active lifetime.

In an embodiment, the total amount of starch hydrolysate additionally contains from 1 to 10 weight percent of maltose. This maltose preferably also is enzymatically converted into glucose. Optionally, the enzyme selected for addition is effective for conversion of both isomaltose and maltose into glucose. Alternatively, an additional enzyme that is different from the isomaltose enzyme may also be introduced to convert maltose into glucose.

The enzyme may be any enzyme that is more effective for depolymerizing isomers such as maltose having a 1,4 ether linkage. A preferred enzyme for acting on isomers having 1,4 linkages is glucoamylase. Optionally, the same or different enzymes may be introduced to covert other oligomeric polysaccharides that may be present in the starch hydrolysate into glucose.

The remaining portion of the total amount of starch hydrolysate containing 80-98 weight percent of glucose is fed into the fermentation broth batch to maintain a glucose concentration of from 5 to 15 g/L in the fermentation broth throughout the feeding step. It has been found that at concentrations higher than about 15 g/L, the isomaltose enzyme is more and more inhibited in activity as glucose concentration is increased.

In an embodiment of the present invention, the remaining portion of the total amount of starch hydrolysate added is provided as an addition composition having a solids content of from 25 to 70 weight percent. Optionally, this remaining portion is added as an addition composition having a solids content of from about 25 to about 45 weight percent, or as an addition composition having a solids content of from about 30 to about 40 weight percent, or as an addition composition having a solids content of from about 45 to about 70 weight percent.

The step of feeding the remaining portion of the total amount of starch hydrolysate into the fermentation broth is more preferably carried out to maintain a glucose concentration of from about 8 to about 12 g/L in the fermentation broth throughout the feeding step. This feeding step maximizes the amount of fermentation product that can be produced in a single batch operation, and minimizes the amount of enzyme required by most efficiently using the organisms and enzymes that are charged to the fermentation vessel.

The remaining portion of the total amount of starch hydrolysate may be added in at least three sub-portion addition steps Optionally, the remaining portion of the total amount of starch hydrolysate is added to the fermentation broth using a variable rate addition system. This system is preferred because it does not introduce temporarily high concentrations of glucose in the fermentation batch that would adversely affect the activity of the enzyme. Examples of such systems include a variable speed pump or a metering valve (such as a throttle valve) operably connected to a pump, which pump or valve can be utilized to vary the amount of hydrolysate introduced into the fermentation broth over time. Optionally, the starch hydrolysate addition is carried out over a time period of from about 4 to about 30 hours, for example from about 5 to about 24 hours, and in some cases to optimize product yield, enzyme usage and production costs, from about 10 to about 20 hours.

In an embodiment, during the addition of the remaining portion of the starch hydrolysate the glucose concentration is monitored by a real-time monitoring system. Real-time monitoring systems include systems that directly monitor glucose concentration and systems that indirectly monitor glucose concentration. Examples of real-time monitoring systems that typically directly monitor glucose concentration include systems based on infrared (IR) spectroscopy, near-infrared (NIR) spectroscopy systems, Fourier transform infrared (FTIR) systems, systems based on refractive index, automated enzyme based measurement systems such as a YSI 2950 Biochemistry Analyzer sold by YSI Life Sciences systems, high performance liquid chromatography (HPLC) based systems, gas chromatography (GC) based systems, and other real-time monitorying systems known to one of skill in the art.

Additionally real-time monitoring systems that indirectly monitor/measure the glucose concentration of a fermentation process can be developed by determining the typical carbon distribution in a particular fermentation process and correlating the glucose concentration present in the fermentation broth to another parameter exhibited by the fermenation, such as, for example, a correlation of the glucose level present in the fermentation broth with a measurement of the carbon dioxide evolution rate and the amount of carbon dioxide present in an off-gas stream from the fermentation vessel. The carbon dioxide can be readily measured through use of a mass spectrometer or other suitable instrumental technique for measuring the components of the off-gas stream. In a preferred embodiment, the glucose concentration is monitored by a real-time monitoring system using infrared spectroscopy. Optionally, the glucose concentration is monitored by a real-time monitoring system using near-infrared spectroscopy. In a particularly preferred aspect of the invention, the real time monitoring systems interface with equipment that controls the introduction of starch hydrolysate introduced into the fermentation broth to facilitate the maintenance of the glucose concentration in the fermentation broth at the desired concentration.

In another aspect of the invention, the glucose concentration is monitored by real-time monitoring system, and a control system utilizes the value of glucose concentration measured to control the introduction of starch hydrolysate (and optionally enzyme) into the fermentation broth. In this aspect of the invention, the starch hydrolysate utilized has greater than 60 percent by weight glucose concentration based on the total carbohydrate, typically greater than 70 percent by weight glucose concentration, for example, greater than 80 percent by weight glucose concentration based on the total carbohydrate and in many instances greater than 90 percent by weight glucose concentration based on the total carbohydrate.

The addition of the remaining portion of the starch hydrolysate is continued until the desired amount of starch hydrolysate has been added. After the desired amount of starch hydrolysate has been added, the fermentation is typically allowed to proceed until the final fermentation broth is produced. Preferably, after the desired amount of starch hydrolysate has been added, the fermentation is allowed to proceed until the glucose concentration is at a concentration below 5 g/L, or preferably below 1 g/L, or more preferably below 0.5 g/L based on the volume of the final fermentation broth.

In an embodiment, the final fermentation broth comprises no more than 3 g/L of isomaltose. Optionally, the final fermentation broth comprises no more than about 1 g/L of isomaltose, and preferably no more than about 0.5 g/L isomaltose.

The fermentation product may be any product that can be prepared by fermenting glucose. In an embodiment, the fermentation product is selected from the group consisting of: amino acids, organic acids, alcohols, diols, polyols, fatty acids, monacyl glycerides, diacyl glycerides, triacyl glycerides, and mixtures thereof. Preferred fermentation products are organic acids, amino acids and their salts thereof, and especially where the organic acid is selected from the group consisting of hydroxyl carboxylic acids, dicarboxylic acids, and tricarboxylic acids and mixtures thereof. Examples of fermentation products that are prepared by the present process are organic acids or amino acids such as lactic acid, citric acid, malonic acid, hydroxy butyric acid, adipic acid, lysine, keto-glutaric acid, glutaric acid, 3-hydroxy-proprionic acid, succinic acid, malic acid, fumaric acid, itaconic acid, muconic acid, methacrylic acid, and acetic acid and derivatives thereof and salts thereof.

The activity of enzymes capable of converting isomaltose and other oligomeric polysaccharide(s) into glucose (such as transglucosidase (TG)), often is at least partially dependent on the pH and composition (including cation composition) of the fermentation broth being utilized. When the fermentation products comprise a carboxylic acid (including hydroxy carboxylic acid(s) and amino acids), it may be desirable to adjust the pH of the fermentation broth to enhance the activity of the enzyme. When desired, a basic compound typically would be added to the fermentation broth to adjust the pH of the broth and therefore enhance the activity of an enzyme, such as a TG enzyme. Basic compounds that may be utilized include compounds having the following cations: ammonium, sodium, potasium, and mixtures thereof.

When the fermentation process utilizes a gypsum recovery process, as described below, the preferred cation utilized comprises Calcium. In a gypsum recovery process, an organic acid fermetation product (including a hydroxy carboxylic acid) is made. During the fermentation, the pH of the fermentation broth is maintained at a desireable level using calcium hydroxide, often used in the form of lime. Once the fermenation is complete, it is desirable to enhance the recovery of the organic acid by adding sulfuric acid to acidulate the broth and thereby increase the percentage of acid that is recovered from the broth. The ions from the lime and sulfuric acid react to form calcium sulfate (gypsum), which can then be readily removed from the fermenation broth by precipitation.

Alternatively, ammonia or ammonium hydroxide is economical and abundant. Ammonium hydroxide has the advantage of providing not only pH buffering, but also supplies an additional bio-available nitrogen source to the fermentation. Examples of fermentations that employ ammonium hydroxide for pH control include fermentation processes for the manufacture of citric acid, lysine or other amino acids. Additionally, ammonium hydroxide is commonly employed in fermentation processes that typically incorporate the ammonium into the fermentation product (such as amino acids) or biomass (such as amino acids or citric acid and other processes known to one of skill in the art), can tolerate ammonium in the final product specifications or processes where the fermentation product is typically recovered through the use of ion exchange, crystallization or liquid-liquid extraction (such as citric acid, succinic acid, or fumaric acid, and other processes known to one of skill art).

Sodium hydroxide and potasium hydroxide are readily available. They typically would be utilized in fermentation processes that require only relatively minor pH control during the fermenation.

The fermentation product is recovered from the fermentation broth. The manner of accomplishing this will depend on the particular product. However, in general, the organism is separated from the liquid phase, typically via a filtration step or centrifugation step, and the product recovered via, for example, distillation, extraction, crystallization, membrane separation, osmosis, reverse osmosis, or other suitable technique. Organic acids typically will require that the fluid containing the organic acid (and salts thereof) be acidulated using acids such as sulfuric acid to recover the organic acid.

Optionally, the amount of enzyme that is used in the fermentation batch is substantially reduced as compared to a like process where all of the starch hydrolysate is charged to the fermentation broth at the beginning of fermentation and enzyme is added after the glucose concentration is reduced to a desired level (as described in US Patent Application Publication 2009/0011481). It has surprisingly been discovered that by holding back a portion of the total starch hydrolysate to be fermented until after the first portion is fermented to the desired glucose concentration level and enzyme is added, and then adding the remainder of the starch hydrolysate in manner to control the glucose concentration to a desired level, one can use substantially less enzyme overall. Optionally, the amount of enzyme used in the present batch process having portion of starch hydrolysate added after the enzyme is at least 25% less than the amount used in a like process having all of the starch hydrolysate added as an initial charge. Optionally, the amount of enzyme used in the present batch process having portion of starch hydrolysate added after the enzyme is at least 40% less than the amount used in a like process having all of the starch hydrolysate added as an initial charge. Optionally, the amount of enzyme used in the present batch process having portion of starch hydrolysate added after the enzyme is at least 50% less than the amount used in a like process having all of the starch hydrolysate added as an initial charge. Optionally, the amount of enzyme to be added is calculated based on the amount of isomaltose in the total amount of starch hydrolysate and the activity of the enzyme when used in a fermentation broth that contains 20 g/L or less of glucose.

The present process provides the ability to make fermentation products on a production scale level with excellent yields and purity. Optionally, the batch process is carried out in to produce batches of at least 25,000 gallons of final fermentation broth.

The present disclosure describes specific aspects, such as preferred amounts of glucose content and enzyme addition conditions. Combinations of separately discussed aspects with each other are specifically contemplated.

### EXAMPLES

Representative embodiments of the present invention will now be described with reference to the following examples that illustrate the principles and practice of the present invention.

The transglucosidase used is based on the TG-L2000 product available from DuPont Biosciences, with the following differences: 1) the final glycerol composition is less than 1%; 2) traces of NaCl are present in the product but <0.1%; 3) the following additional preservatives and stabilizers are added - Glucose (20- 30% w/w) and sodium benzoate (0.2-0.4% w/w).

The fermentation broth consists of water as well as sufficient nutrients required to enable cell growth. The fermentation broth also contains an anti-foam agent to control foaming in the fermentation.

A fermentor is partially filled to 65% of volumetric operating capacity with the components of the fermentation broth as well as 60% of the total glucose to be added. The glucose source is a starch hydrolysate of which 95% of the carbohydrates are glucose. The temperature is controlled at a value that is suitable for the fermentation host organism, in this case 34° C. The fermentor is then inoculated with a suitable fermentation microorganism, in this case, a yeast engineered for the production of lactic acid. The pH is maintained above 3.1 by the addition of calcium hydroxide.

As the fermentation proceeds, the glucose is converted into lactic acid at a certain yield. The glucose concentration is monitored by a near infrared spectroscopy system (NIR) (Brueker - Matrix F model). When the glucose has been reduced to less than 20 g/L as measured by NIR, the transglucosidase enzyme as described above is added at a dose of 0.025% (volume enzyme solution/volume fermentation broth).

The fermentation is then allowed to further proceed until the glucose concentration reaches 10 g/L. The remaining 40% of the total glucose is then gradually added to the fermentor at a rate controlled to maintain the glucose between 8 g/L and 12 g/L as measured by the NIR.

Once 100% of the target dextrose has been added, the glucose feed is stopped and the fermentation proceeds until the glucose is reduced below 0.5 g/L. Unless otherwise indicated, all parts and percentages are by weight and all molecular weights are weight average molecular weights.

## Claims

1. A batch fermentation process for fermenting a starch hydrolysate containing 80-98 weight percent of glucose based on total carbohydrate and 0.3-5% weight percent of isomaltose based on total carbohydrate to a fermentation product, comprising, in order:
a) forming a fermentation broth containing a first portion of a total amount of the starch hydrolysate so that the fermentation broth has an initial glucose concentration of at least 50 g/L;
b) fermenting the starch hydrolysate in the fermentation broth in an initial fermentation step to produce a fermentation product until the fermentation broth contains 30 g/L or less of glucose;
c) adding to the fermentation broth an effective amount of at least one active enzyme that converts isomaltose into glucose;
d) feeding the remaining portion of the total amount of starch hydrolysate containing 80-98 weight percent of glucose based on total carbohydrate and 0.3-5% weight percent of isomaltose based on total carbohydrate into the fermentation broth to maintain a glucose concentration of from 5 to 5 g/L in the fermentation broth throughout the feeding step; and
e) producing a final fermentation broth containing the fermentation product.

2. The fermentation process of any of the preceding claims, wherein the starch hydrolysate added in step d) is provided as an addition composition having a solids content of from 25 to 70 weight percent.

3. The fermentation process of any of the preceding claims, wherein the first portion of the total amount of the starch hydrolysate contains from 50 to 80 percent of the total amount of starch hydrolysate to be added to the fermentation broth.

4. The fermentation process of any of the preceding claims, wherein the total amount of starch hydrolysate additionally contains from 1 to 10 weight percent of maltose.

5. The fermentation process of any of the preceding claims, wherein the final fermentation broth comprises no more than 3 g/L of isomaltose.

6. The fermentation process of any of the preceding claims, wherein the final fermentation broth comprises no more than 5 g/L of glucose.

7. The fermentation process of any of the preceding claims, wherein during step d), the glucose concentration is monitored by a real-time monitoring system.

8. The fermentation process of any of the preceding claims, wherein during step d), the glucose concentration is monitored by a real-time monitoring system using infrared spectroscopy.

9. The fermentation process of any of the preceding claims, wherein the active enzyme is added to the fermentation broth in a single addition step metered over a time of from 3 minutes to 3 hours.

10. The fermentation process of any of the preceding claims, wherein during the feeding step, the remaining portion of the total amount of starch hydrolysate is fed into the fermentation broth using a variable rate addition system.

11. The fermentation process of any of the preceding claims, wherein the fermentation is carried out using yeast.

12. The fermentation process of any of the preceding claims, wherein the fermentation is carried out using bacteria.

13. The fermentation process of any of the preceding claims, wherein the fermentation product is selected from the group consisting of: amino acids, organic acids, alcohols, polyols, fatty acids, monacyl glycerides, diacyl glycerides, triacyl glycerides, and mixtures thereof.

14. The fermentation process of any of the preceding claims, wherein the glucose concentration of the fermentation broth containing a first portion of a total amount of the starch hydrolysate of step a) is from 50 g/L to 150 g/L.

15. A batch fermentation process for fermenting a starch hydrolysate containing at least 60 weight percent of glucose based on total carbohydrate and 0.3-5% weight percent of isomaltose based on total carbohydrate to a fermentation product, comprising, in order:
a) forming a fermentation broth containing a first portion of a total amount of the starch hydrolysate so that the fermentation broth has an initial glucose concentration of at least 50 g/L;
b) fermenting the starch hydrolysate in the fermentation broth in an initial fermentation step to produce a fermentation product until the fermentation broth contains 30 g/L or less of glucose;
c) adding to the fermentation broth an effective amount of at least one active enzyme that converts isomaltose into glucose;
d) feeding the remaining portion of the total amount of starch hydrolysate containing at least 60 weight percent of glucose based on total carbohydrate and 0.3-5% weight percent of isomaltose based on total carbohydrate into the fermentation broth to maintain a glucose concentration of from 5 to 15 g/L in the fermentation broth throughout the feeding step; and
e) producing a final fermentation broth containing the fermentation product,
wherein during step (d) the glucose concentration is monitored by a real-time monitoring system, and wherein the real-time monitoring system interfaces with equipment that controls the introduction of starch hydrolysate in step (d).

## Patentansprüche

1. Batchfermentationsverfahren zum Fermentieren eines Stärkehydrolysats, enthaltend 80-98 Gew.-% Glukose basierend auf dem Gesamtkohlenhydrat und 0,3-5 Gew.-% Isomaltose basierend auf dem Gesamtkohlenhydrat, zu einem Fermentationsprodukt, umfassend in der folgenden Reihenfolge:
a) Bilden einer Fermentationsbrühe, enthaltend einen ersten Teil einer Gesamtmenge des Stärkehydrolysats, sodass die Fermentationsbrühe eine anfängliche Glukosekonzentration von mindestens 50 g/l aufweist;
b) Fermentieren des Stärkehydrolysats in der Fermentationsbrühe in einem ersten Fermentationsschritt, um ein Fermentationsprodukt zu erzeugen, bis die Fermentationsbrühe 30 g/l oder weniger Glukose enthält;
c) Zugeben einer wirksamen Menge mindestens eines aktiven Enzyms, das Isomaltose in Glukose umwandelt, zu der Fermentationsbrühe;
d) Zuführen des verbliebenen Teils der Gesamtmenge des Stärkehydrolysats, das 80-98 Gew.-% Glukose basierend auf dem Gesamtkohlenhydrat und 0,3-5 Gew.-% Isomaltose basierend auf dem Gesamtkohlenhydrat enthält, in die Fermentationsbrühe, um während des Zuführschritts eine Glukosekonzentration von 5 bis 15 g/l in der Fermentationsbrühe aufrechtzuerhalten; und
e) Erzeugen einer finalen Fermentationsbrühe, die das Fermentationsprodukt enthält.

2. Fermentationsverfahren nach einem der vorhergehenden Ansprüche, wobei das in Schritt d) zugegebene Stärkehydrolysat als eine zusätzliche Zusammensetzung mit einem Feststoffgehalt von 25 bis 70 Gew.-% bereitgestellt wird.

3. Fermentationsverfahren nach einem der vorhergehenden Ansprüche, wobei der erste Teil der Gesamtmenge des Stärkehydrolysats von 50 bis 80 % der zu der Fermentationsbrühe zu gebenden Gesamtmenge des Stärkehydrolysats enthält.

4. Fermentationsverfahren nach einem der vorhergehenden Ansprüche, wobei die Gesamtmenge Stärkehydrolysat zusätzlich von 1 bis 10 Gew.-% Maltose enthält.

5. Fermentationsverfahren nach einem der vorhergehenden Ansprüche, wobei die finale Fermentationsbrühe nicht mehr als 3 g/l Isomaltose umfasst.

6. Fermentationsverfahren nach einem der vorhergehenden Ansprüche, wobei die finale Fermentationsbrühe nicht mehr als 5 g/l Glukose umfasst.

7. Fermentationsverfahren nach einem der vorhergehenden Ansprüche, wobei die Glukosekonzentration während Schritt d) durch ein Echtzeit-Überwachungssystem überwacht wird.

8. Fermentationsverfahren nach einem der vorhergehenden Ansprüche, wobei die Glukosekonzentration während Schritt d) durch ein Echtzeit-Überwachungssystem unter Verwendung von Infrarotspektroskopie überwacht wird.

9. Fermentationsverfahren nach einem der vorhergehenden Ansprüche, wobei das aktive Enzym in einem einzigen Zugabeschritt zu der Fermentationsbrühe gegeben wird und über einen Zeitraum von 3 Minuten bis 3 Stunden zudosiert wird.

10. Fermentationsverfahren nach einem der vorhergehenden Ansprüche, wobei der verbliebene Teil der Gesamtmenge Stärkehydrolysat während des Zuführschritts unter Verwendung eines Zugabesystems mit variabler Geschwindigkeit der Fermentationsbrühe zugeführt wird.

11. Fermentationsverfahren nach einem der vorhergehenden Ansprüche, wobei die Fermentation unter Verwendung von Hefe durchgeführt wird.

12. Fermentationsverfahren nach einem der vorhergehenden Ansprüche, wobei die Fermentation unter Verwendung von Bakterien durchgeführt wird.

13. Fermentationsverfahren nach einem der vorhergehenden Ansprüche, wobei das Fermentationsprodukt aus der Gruppe bestehend aus Aminosäuren, organischen Säuren, Alkoholen, Polyolen, Fettsäuren, Monoacylglyceriden, Diacylglyceriden, Triacylglyceriden und Mischungen davon ausgewählt ist.

14. Fermentationsverfahren nach einem der vorhergehenden Ansprüche, wobei die Glukosekonzentration der Fermentationsbrühe von Schritt a), die einen ersten Teil der Gesamtmenge des Stärkehydrolysats enthält, von 50 g/l bis 150 g/l beträgt.

15. Batchfermentationsverfahren zum Fermentieren eines Stärkehydrolysats, enthaltend mindestens 60 Gew.-% Glukose basierend auf dem Gesamtkohlenhydrat und 0,3-5 Gew.-% Isomaltose basierend auf dem Gesamtkohlenhydrat, zu einem Fermentationsprodukt, umfassend in der folgenden Reihenfolge:
a) Bilden einer Fermentationsbrühe, enthaltend einen ersten Teil einer Gesamtmenge des Stärkehydrolysats, sodass die Fermentationsbrühe eine anfängliche Glukosekonzentration von mindestens 50 g/l aufweist;
b) Fermentieren des Stärkehydrolysats in der Fermentationsbrühe in einem ersten Fermentationsschritt, um ein Fermentationsprodukt zu erzeugen, bis die Fermentationsbrühe 30 g/l oder weniger Glukose enthält;
c) Zugeben einer wirksamen Menge mindestens eines aktiven Enzyms, das Isomaltose in Glukose umwandelt, zu der Fermentationsbrühe;
d) Zuführen des verbliebenen Teils der Gesamtmenge des Stärkehydrolysats, das mindestens 60 Gew.-% Glukose basierend auf dem Gesamtkohlenhydrat und 0,3-5 Gew.-% Isomaltose basierend auf dem Gesamtkohlenhydrat enthält, in die Fermentationsbrühe, um während des Zuführschritts eine Glukosekonzentration von 5 bis 15 g/l in der Fermentationsbrühe aufrechtzuerhalten; und
e) Erzeugen einer finalen Fermentationsbrühe, die das Fermentationsprodukt enthält,
wobei die Glukosekonzentration während Schritt (d) mittels eines Echtzeit-Überwachungssystems überwacht wird und wobei das Echtzeit-Überwachungssystem eine Schnittstelle mit einem Gerät aufweist, welches die Einführung des Stärkehydrolysats in Schritt (d) steuert.

## Revendications

1. Procédé de fermentation par lots pour faire fermenter un hydrolysat d'amidon contenant 80-98 pour cent en poids de glucose sur la base des glucides totaux et 0,3-5 pour cent en poids d'isomaltose sur la base des glucides totaux en un produit de fermentation, comprenant, dans l'ordre :
a) former un bouillon de fermentation contenant une première partie d'une quantité totale de l'hydrolysat d'amidon de telle sorte que le bouillon de fermentation a une concentration initiale en glucose d'au moins 50 g/L ;
b) faire fermenter l'hydrolysat d'amidon dans le bouillon de fermentation dans une étape de fermentation initiale pour produire un produit de fermentation jusqu'à ce que le bouillon de fermentation contienne 30 g/L ou moins de glucose ;
c) ajouter au bouillon de fermentation une quantité efficace d'au moins une enzyme active qui convertit l'isomaltose en glucose ;
d) introduire la partie restante de la quantité totale d'hydrolysat d'amidon contenant 80-98 pour cent en poids de glucose sur la base des glucides totaux et 0,3-5 pour cent en poids d'isomaltose sur la base des glucides totaux dans le bouillon de fermentation pour maintenir une concentration en glucose de 5 à 15 g/L dans le bouillon de fermentation dans toute l'étape d'introduction ; et
e) produire un bouillon de fermentation final contenant le produit de fermentation.

2. Procédé de fermentation selon l'une quelconque des revendications précédentes, dans lequel l'hydrolysat d'amidon ajouté à l'étape d) est fourni en tant que composition d'addition ayant une teneur en matières solides de 25 à 70 pour cent en poids.

3. Procédé de fermentation selon l'une quelconque des revendications précédentes, dans lequel la première partie de la quantité totale de l'hydrolysat d'amidon contient de 50 à 80 pour cent de la quantité totale d'hydrolysat d'amidon à ajouter au bouillon de fermentation.

4. Procédé de fermentation selon l'une quelconque des revendications précédentes, dans lequel la quantité totale d'hydrolysat d'amidon contient en outre de 1 à 10 pour cent en poids de maltose.

5. Procédé de fermentation selon l'une quelconque des revendications précédentes, dans lequel le bouillon de fermentation final ne comprend pas plus de 3 g/L d'isomaltose.

6. Procédé de fermentation selon l'une quelconque des revendications précédentes, dans lequel le bouillon de fermentation final ne comprend pas plus de 5 g/L de glucose.

7. Procédé de fermentation selon l'une quelconque des revendications précédentes, dans lequel, pendant l'étape d), la concentration en glucose est surveillée par un système de surveillance en temps réel.

8. Procédé de fermentation selon l'une quelconque des revendications précédentes, dans lequel, pendant l'étape d), la concentration en glucose est surveillée par un système de surveillance en temps réel utilisant la spectroscopie infrarouge.

9. Procédé de fermentation selon l'une quelconque des revendications précédentes, dans lequel l'enzyme active est ajoutée au bouillon de fermentation en une seule étape d'addition unique mesurée au cours d'un temps de 3 minutes à 3 heures.

10. Procédé de fermentation selon l'une quelconque des revendications précédentes, dans lequel, pendant l'étape d'introduction, la partie restante de la quantité totale d'hydrolysat d'amidon est introduite dans le bouillon de fermentation à l'aide d'un système d'addition à vitesse variable.

11. Procédé de fermentation selon l'une quelconque des revendications précédentes, dans lequel la fermentation est effectuée à l'aide de levure.

12. Procédé de fermentation selon l'une quelconque des revendications précédentes, dans lequel la fermentation est effectuée à l'aide de bactéries.

13. Procédé de fermentation selon l'une quelconque des revendications précédentes, dans lequel le produit de fermentation est choisi dans le groupe consistant en : acides aminés, acides organiques, alcools, polyols, acides gras, monoacyl glycérides, diacyl glycérides, triacyl glycérides et leurs mélanges.

14. Procédé de fermentation selon l'une quelconque des revendications précédentes, dans lequel la concentration en glucose du bouillon de fermentation contenant une première partie d'une quantité totale de l'hydrolysat d'amidon de l'étape a) est de 50 g/L à 150 g/L.

15. Procédé de fermentation par lots pour faire fermenter un hydrolysat d'amidon contenant au moins 60 pour cent en poids de glucose sur la base des glucides totaux et 0,3-5 pour cent en poids d'isomaltose sur la base des glucides totaux en un produit de fermentation comprenant, dans l'ordre :
a) former un bouillon de fermentation contenant une première partie d'une quantité totale de l'hydrolysat d'amidon de telle sorte que le bouillon de fermentation a une concentration initiale en glucose d'au moins 50 g/L ;
b) faire fermenter l'hydrolysat d'amidon dans le bouillon de fermentation dans une étape de fermentation initiale pour produire un produit de fermentation jusqu'à ce que le bouillon de fermentation contienne 30 g/L ou moins de glucose ;
c) ajouter au bouillon de fermentation une quantité efficace d'au moins une enzyme active qui convertit l'isomaltose en glucose ;
d) introduire la partie restante de la quantité totale d'hydrolysat d'amidon contenant au moins 60 pour cent en poids de glucose sur la base des glucides totaux et 0,3-5 pour cent en poids d'isomaltose sur la base des glucides totaux dans le bouillon de fermentation pour maintenir une concentration en glucose de 5 à 15 g/L dans le bouillon de fermentation dans toute l'étape d'introduction ; et
e) produire un bouillon de fermentation final contenant le produit de fermentation,
dans lequel, pendant l'étape (d), la concentration en glucose est surveillée par un système de surveillance en temps réel, et dans lequel le système de surveillance en temps réel s'interface avec un équipement qui commande l'introduction de l'hydrolysat d'amidon dans l'étape (d).
